# EUROPEAN PATENT SPECIFICATION

(11) **EP 2 266 887 B1**
(45) Date of publication and mention of the grant of the patent: **18.12.2013**
(21) Application number: 09731454.6
(22) Date of filing: 31.03.2009
(51) Int. Cl.: B65D 41/18, A61J 1/05, B65D 1/02, A61F 9/00

(54) **LIQUID CONTAINER**
FLÜSSIGKEITSBEHÄLTER
RÉCIPIENT À LIQUIDE

(30) Priority: 08.04.2008 JP 2008002196 U
(43) Date of publication of application: 29.12.2010
(73) Proprietor: Rohto Pharmaceutical Co., Ltd., Osaka-shi Osaka 544-8666 (JP)
(72) Inventor: KOKUBO, Shigehiko, Osaka-shi Osaka 544-8666 (JP)
(74) Representative: Laufhütte, Dieter
(86) International application number: PCT/JP2009/056615
(87) International publication number: WO 2009/125692

(56) References cited:
- FR-A1- 2 789 365
- JP-A- 10 329 855
- JP-A- 10 329 855
- JP-U- 54 073 139
- JP-U- 61 169 045

## Description

### TECHNICAL FIELD

The present invention relates to a liquid container useful as a container for an eye drop (ophthalmic solution), a nose drop (nasal solution), a contact-lens cleaning solution or the like.

### BACKGROUND ART

Heretofore, as a container for an eye drop or the like, there has been commonly known a type which comprises a container body having a nozzle, and a cap detachably attachable to the container body, wherein it is designed to allow a medical solution to be instilled (dropped) from a tip end of the nozzle through an operation of gently pressing the container body while orienting the tip end of the nozzle downwardly under a condition that the cap is detached.

Particularly, a so-called twist cap-type liquid container having a pair of lockable arms provided on an inner peripheral surface of a cap is widely used as the container for an eye drop or the like, wherein it is designed such that the cap is attached to a container body by locking the lockable arms onto a locking protrusion of the container body, and then the cap is detached by releasing the locked state between the container body and each of the lockable arms through an operation of twisting the cap to bring the cap into riding contact with a shoulder portion (a portion between a liquid storage portion and a tubular neck portion holding a nozzle) of the container body, as described in the following Patent Document 1, which discloses the preamble features of claim 1.

While the twist cap-type liquid container is capable of obtaining a stronger clicking feeling when the lockable arms are engaged with the locking protrusion, to allow a user to check an attached state of the cap based on the clicking feeling, a sub-type designed to obtain stronger clicking feelings is also widely used, which has a protrusion provided on the container body, e.g., on a top end of the shoulder portion, in addition to the aforementioned locking protrusion, and a corresponding protrusion provided on an inner surface of the cap, wherein a clicking feeling is obtained when the protrusion of the cap is moved to cross over the protrusion of the container body during a cap attaching operation.

As a production method for this sub-type of liquid container (container body), there have been commonly known a direct blow molding process designed to receive a resin material in a die to form the resin material directly into a final product shape by blow molding, and an injection blow molding process designed to form a pre-molded product by injection molding and further subject the pre-molded product to blow molding to obtain a final product. In many cases, the liquid container has been produced by the direct blow molding process so far. This is primarily because the direct blow molding process is advantageous in view of equipment, such as a smaller number of dies, i.e., low cost, and a capability to make dies in a short period.

However, recent years, there is a tendency for requirements to mass-produce containers stable in accuracy, and thereby a liquid container is increasingly produced by the injection blow molding process including an injection step. In reality, this involves the following problem. Specifically, in the injection blow molding process, it is necessary to provide a handing circular tube portion in a molded product, due to a need for handling the pre-molded product to transfer it to a blow-molding die, in a course of the molding. In this case, it is considered that it is optimal to provide the handling circular tube portion at a position corresponding to a portion just above the liquid storage portion, i.e., the top end of the shoulder portion, in view of stably performing the handling. Thus, in the injection blow molding process, it is difficult to provide a protrusion on the shoulder portion. Therefore, for practical purposes, the sub-type of liquid container designed to enhance a clicking feeling cannot respond to production based on the injection blow molding process.
Patent Document 1: JP 10-329855A
FR 2 789 365 A1 , JP 54 073139 U, JP 61 169045 U disclose reinforcing ribs arranged at lockable pieces of respective closures. JP 61 169045 U discloses the provision of two pairs of ribs, with two ribs on one respective lockable piece. These pairs of ribs are symmetrically arranged at acute angles on either side and they are not perpendicular to each other.

### DISCLOSURE OF THE INVENTION

The present invention has been made in view of the above circumstances, and an object thereof is to provide a liquid container capable of obtaining a strong clicking feeling during a cap attaching operation and responding to production based on an injection blow molding process.

In order to achieve this object, a liquid container according to the present invention comprises the features of claim 1.

In this liquid container, when the cap is attached along the axial direction of the tubular neck portion of the container body, the nozzle and the tubular neck portion are inserted between the pair of lockable arms provided on the inner top region of the cap. Then, when the cap is pushed to a position where an edge of an opening of the cap comes into contact with the container body, the respective engagement portions of the lockable arms are engaged with the locking protrusion, and thereby the cap is locked with respect to the container body. During the operation of pushing the cap, when the engagement portions is moved to cross over the locking protrusion, the lockable arms are bent (elastically deformed) and then elastically returned to allow a clicking feeling to be obtained. In the above container, respective rigidities of the lockable arms are increased by the first and the second reinforcing ribs in the above manner, so that, when the engagement portions cross over the locking protrusion, the lockable arms are elastically returned significantly strongly, which makes it possible to obtain a significantly strong clicking feeling. Thus, based on only a clicking feeling produced when the lockable arms are engaged with the locking protrusion, a significantly strong clicking feeling can be obtained at a level equivalent to that in the conventional liquid container designed to obtain a strong clicking feeling by providing a protrusion on the shoulder portion.

Particularly, in addition to the first reinforcing ribs each adapted to restrict a flexural displacement of a respective one of the lockable arms in the arrangement direction of the lockable arms, the second reinforcing ribs each connecting a respective one of the lockable arms and an inner surface of the cap are provided. This makes it possible to efficiently increase the rigidity of each of the lockable arms by utilizing a narrow internal space of the cap.

Preferably, each of the engagement portions is provided in a distal end region of a corresponding one of the lockable arms, and each of the first reinforcing ribs is formed over a region from a base end approximately to a distal end of a corresponding one of the lockable arms, wherein a width of the first reinforcing rib in a direction perpendicular to a thicknesswise direction of the first reinforcing rib decreases in a direction from the base end to the distal end. In this case, it is preferable that each of the second reinforcing rib is provided in a vicinity of the base end of a corresponding one of the lockable arms.

This structure makes it possible to adequately permit a flexural (bending) deformation in the distal end region (portion provided with the engagement portion) of each of the lockable arms while increasing the rigidity of the entire lockable arm, so that the lockable arm can be adequately locked to the locking protrusion while being elastically deformed along with pushing of the cap, and a clicking feeling to be produced during the locking can be effectively enhanced.

### BRIEF DESCRIPTION OF THE DRAWINGS

FIG. 1 is a schematic sectional view showing a substantial part of a liquid container according to the present invention (wherein FIG. 1(a) and FIG. 1(b) illustrate a state in a course of a cap attaching operation, and a cap-attached state, respectively).
FIG. 2 is a sectional view showing a substantial part of the liquid container, taken along the line II-II in FIG. 1(b).
FIG. 3 is a sectional view showing a substantial part of the liquid container, taken along the line III-III in FIG. 1(b).
FIG. 4 is an explanatory diagram illustrating one example of a production method for a container body (except a nozzle).

### BEST MODE FOR CARRYING OUT THE INVENTION

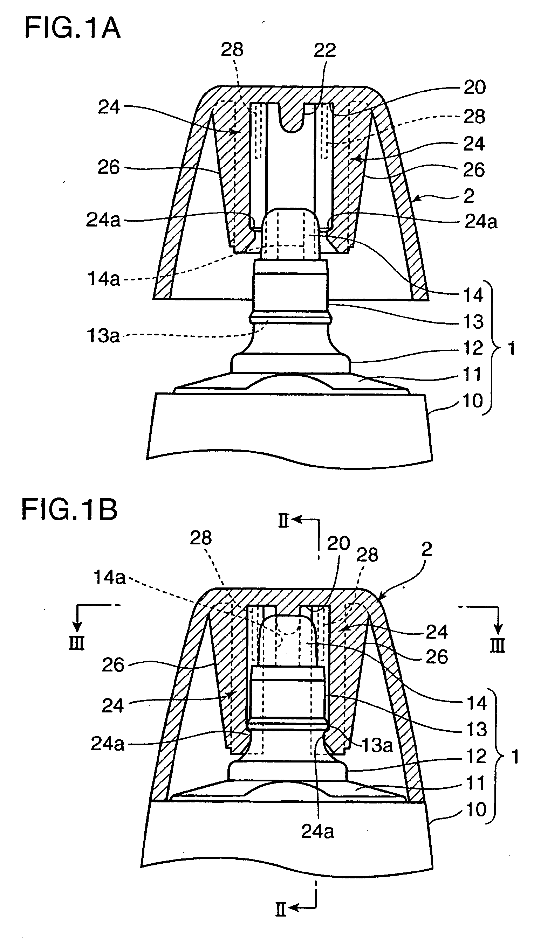

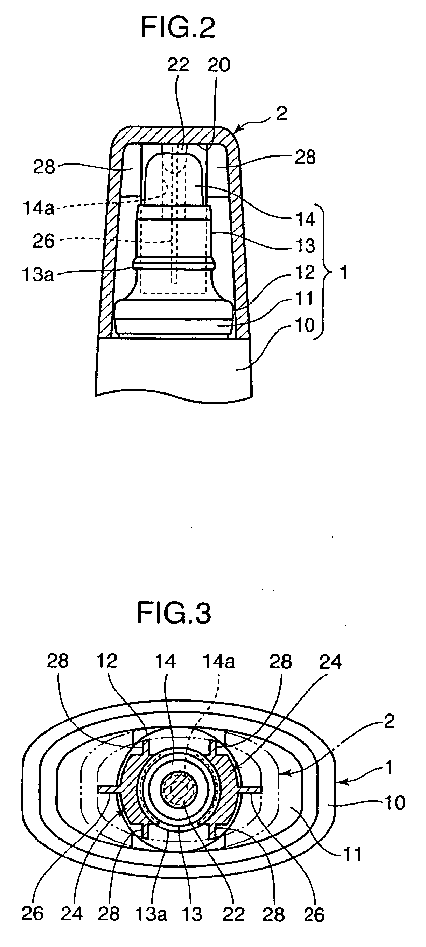

FIGS. 1 and 2 illustrate a substantial part of a liquid container according to the present invention.

The liquid container (hereinafter abbreviated as "container") illustrated in FIGS. 1 and 2 is a so-called twist cap-type container. In this embodiment, this container is an eye drop container which comprises a container body 1 having a nozzle 14, and a cap 2 detachably attachable to the container body 1 to cover the nozzle 14, wherein it is designed, during instillation, to allow a medical solution contained in the container body 1 to be instilled from a tip end of the nozzle through an operation of gently pressing the container body 1 while orienting the tip end of the nozzle downwardly under a condition that the cap 2 is detached.

As shown in FIGS. 2 and 3, the container body 1 is formed in a flat shape in a forward-rearward direction (upward-downward direction in FIG. 3). The container body 1 has a liquid storage portion 10 storing an eye drop, a shoulder portion 11 continuous with a top end of the liquid storage portion 10, and a circular tubular neck portion 13 provided to stand in a central position of the shoulder portion 11 through a circular tube portion 12, wherein a portion other than the nozzle 14 is integrally molded using a synthetic resin, such as polypropylene, polyethylene or polyethylene terephthalate. The container body 1 (a portion thereof other than the nozzle 1) is produced by an after-mentioned injection blow molding process, and the circular tube portion 12 is provided to handle a pre-molded product in a course of the molding.

The nozzle 14 is molded separately from the liquid storage portion 10, using a soft synthetic resin, such as low-density polyethylene, linear low-density polyethylene, high-density polyethylene or polypropylene, and integrated with the container body 1 by press-fitting into the circular tubular neck portion 13.

As shown in FIGS. 1 to 3 (in FIG. 3, only a part of the cap 2 is illustrated by the solid line for the sake of simplicity), the cap 2 is formed in a top-closed tubular shape to cover a region above the shoulder portion 11. Further, it is configured such that, in the state after being attached to the container body 2, a lateral surface of the container body 1 and a lateral surface of the cap 2 form a continuous smooth contour line in an upward-downward direction, as shown in FIGS. 1 to 3. While the cap 2 is made from a synthetic resin, such as polypropylene, polyethylene or polystyrene, as with the container body 1 (the portion thereof other than the nozzle 1), it is formed in a slightly harder structure than the container body 1.

The cap 2 has a plug protrusion 22 provided on a central region of an inner top surface 20 thereof to protrude downwardly, so that, in a state after attaching the cap (cap-attached state), the plug protrusion 22 is inserted in a nozzle hole 14a of the nozzle 14 in close contact relation with an inner peripheral surface of the nozzle hole 14a, and thereby the nozzle hole 14a is sealed.

The cap 2 also has a pair of lockable arms 24 (which is equivalent to "lockable pieces" of the present invention) integrally formed on the inner top surface 20 at respective positions on both sides of the plug protrusion 22.

The lockable arms 24 are provided to extend along an axial direction of the circular tubular neck portion 13, and formed with a pair of hook portions 24a (which is equivalent to "engagement portions" of the present invention) on respective distal end region of opposing surfaces thereof, so that, in the cap-attached state, the nozzle 14 and the circular tubular neck portion 13 are inserted between the lockable arms 24, and the hook portion 24a is engaged with a ring-shaped locking protrusion 13a formed in a vicinity of a base end of the circular tube neck portion 13, whereby the cap 2 is locked to the container body 1 through the lockable arms 24, as shown in FIG. 1(b). Each of the lockable arm 24 has a cross-sectionally arc shape as shown in FIG. 3, and they are provided in such a manner as to surround the circular tube neck portion 13 from laterally opposed directions.

Further, a reinforcing rib for the lockable arms 24 is formed on the inner top surface 20 of the cap 2. Specifically, the cap is provided with a pair of first reinforcing ribs 26 each formed to extend in an upward-downward direction along a central region of a back surface of a respective one of the lockable arms 24 (a surface on an opposite side of a respective one of the opposing surfaces) and adapted to restrict a lateral displacement of a respective one of the lockable arm 24, and a pair of second reinforcing ribs 28 each connecting a respective one of the lockable arms 24 and an inner surface of the cap in a forward-rearward direction.

Each of the first reinforcing ribs 26 is formed to have a small thickness and extend over a region from a base end approximately to a distal end of a respective one of the lockable arms 24, and a width (dimension in the lateral direction) of the first reinforcing rib decreases in a direction from the base end to the distal end. Each of the second reinforcing ribs 28 is formed to have a small thickness and provided in a vicinity of the base end of a respective one of the lockable arms 24.

Meanwhile, the container body 1 (the portion thereof other than the nozzle 14) is produced by a so-called injection blow molding process. This is intended to ensure accuracy of the circular tubular neck portion 13 from a molding stage of the container body 1 to allow post-machining to be omitted.

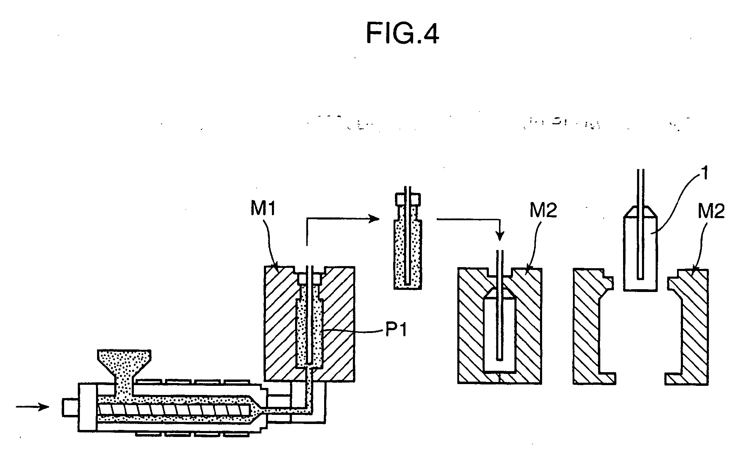

FIG. 4 schematically shows a production method for the container body 1, based on the above molding process. In the production method, a resin material is injected into an injection-molding die M1 to once form a pre-molded product P1 of the container body 1 by injection molding (injection molding step). Then, the pre-molded product P1 is taken out of the die M1, and, after being set in a blow-molding die M2 by a handling apparatus (not shown), subjected to blow molding (blow molding step). In this manner, the container body 1 is produced by accurately forming the circular tubular neck portion 13 through the injection molding, and then blow-molding the liquid storage portion 10. In the injection molding step, the circular tube portion 12 is accurately formed as well as the circular tubular neck portion 13. Then, during an operation of transferring the pre-molded product P1 from the injection-molding die M1 to the blow-molding die M2, the circular tube portion 12 is held by the handling apparatus, to allow the pre-molded product P1 to be transferred stably and adequately.

The container is used in the following manner. Firstly, during instillation, the cap 2 is detached from the container body 1 through an operation of turning the cap 1 from the cap-attached state illustrated in FIG. 1(b), about the circular tubular neck portion 13 by about 90°. Specifically, when the cap 2 is turned, the cap 2 is brought into riding contact with the shoulder portion 11 of the container body 1, and, along with the riding contact, the cap 2 is pushed upwardly, so that the locked state between the circular tubular neck portion 13 and each of the lockable arms 24 is released. Thus, subsequently, the cap 2 can be detached from the container body 1 along the circular tubular neck portion 13 to perform instillation.

Then, after use, the cap 2 is attached along the circular tubular neck portion 13 of the container body 1. Specifically, the cap 2 is pushed along the circular tubular neck portion 13, in such a manner that it is pushingly moved to a position where an edge of an opening of the cap comes into contact with the container body 1. Through this operation, the hook portion 24a of each of the lockable arms 24 is moved to cross over the locking protrusion 13a, and engaged with the locking protrusion 13a, so that the cap 2 is locked to the container body 1.

During the operation of pushing the cap 2, when the lockable arms 24 are moved to cross over the locking protrusion 13a, the lockable arms 24 are once bent (elastically deformed) and then elastically returned to allow a clicking feeling to be obtained, so that, based on the clicking feeling, a user can detect a fact that the cap 2 has been attached. Particularly, in this container, the relatively long lockable arms 24 each extending from the inner top surface 20 of the cap 2 to a vicinity of the base end of the circular tubular neck portion 13 are provided, and further the first and second reinforcing ribs 26, 28 are provided to increase respective rigidities of the lockable arms 24. Therefore, when the hook portions 24a cross over the locking protrusion 13a, the lockable pieces 24 are elastically returned significantly strongly, which makes it possible to obtain a significantly strong clicking feeling. Thus, based on only a clicking feeling produced when the lockable pieces 24 are engaged with the locking protrusion 13, a strong clicking feeling can be obtained at a level equivalent to that in this type of container, i.e., a conventional container designed to enhance a clicking feeling by providing a protrusion on the shoulder portion, etc., in addition to the lockable arms.

Particularly, in this container, in addition to the first reinforcing ribs 26 each adapted to restrict a flexural displacement of a respective one of the lockable pieces 24 in the lateral direction, the second reinforcing ribs 28 each connecting a respective one of the lockable pieces 24 and the inner surface of the cap are provided to efficiently increase the rigidity of each of the lockable pieces by utilizing a narrow internal space of the cap, and each of the first reinforcing ribs 26 is formed such that the width thereof becomes tapered, so that it becomes possible to permit a flexural deformation of the distal end region of each of the lockable arms 24 provided with the hook portions 24a, while increasing the rigidity of the entire lockable arm 24. Thus, the lockable piece 24 can be adequately locked to the locking protrusion 13a by the cap pushing operation, and a clicking feeling to be produced during the locking can be effectively enhanced.

As above, in the liquid container according to the present invention, based on only a clicking feeling produced when the lockable arms 24 are engaged with the locking protrusion 13a, a strong clicking feeling can be obtained at a level equivalent to that in the conventional container having a protrusion provided on the shoulder portion. In other words, a strong clicking feeling can be obtained without providing a protrusion on the top end of the shoulder portion and others, and it becomes possible to easily respond to the injection blow molding process requiring to provide the circular tube portion 12 in the pre-molded product P1 and handle the pre-molded product P1, in a course of molding.

Thus, there is an advantageous effect of being able to obtain a strong clicking feeling during the cap attaching operation, and mass-produce a highly-accurate container body 1 based on the injection blow molding process.

The aforementioned container is one example of a preferred embodiment of the liquid container according to the present invention, and a specific structure thereof may be appropriately changed without departing from the spirits and scope of the present invention.

For example, a specific structure, such as shape, of the nozzle 14, is not limited to those in the above embodiment. Specifically, in addition to a nozzle 14 having a straight shape with an approximately diameter dimension in its entirety as in the above embodiment, a nozzle 14 having a shape tapered in a direction from a base end to a distal end thereof or a shape with an outer peripheral surface thereof provided with a ring-shaped protrusion to enhance a droplet-forming capability may also be used.

Further, a specific structure, such as shape or formation range, of each of the locking protrusion 13a may be appropriately selected to effectively enhance a clicking feeling while allowing the lockable arms 24 to be adequately locked to the locking protrusion 13a along with pushing of the cap 2.

In the above embodiment, the pair of lockable arms 24 arranged in a diametral direction of the circular tubular neck portion 13 are provided on the cap 2 as "lockable pieces" of the present invention. Alternatively, an additional one of the pair of lockable arms 24 may be further provided, wherein the two pairs of, or total four, lockable arms 24, are locked to the circular tubular neck portion 12 from four directions. It is understood that three or more pairs of the lockable pieces (lockable arms 24) may be provided.

In the above embodiment, the circular tubular neck portion 13 having a cross-sectionally circular shape is provided in the container body 1 as "tubular neck portion" of the present invention. Alternatively, a shape of the tubular neck portion may be a polygonal shape, such as a cross-sectionally hexagonal or octagonal shape.

Although the above embodiment has been described based on an example where the liquid container of the present invention is used as an eye drop container, it is understood that the liquid container of the present invention is usable as a container for a liquid other than an eye drop, such as a container for a nose drop or a contact-lens cleaning solution. The molding materials for the container body 1 and the cap 2, mentioned in the description of the above embodiment, are an example of a preferred material for an eye drop container, and a specific molding material for the container body 1 or other element may be appropriately selected depending on an intended purpose of the container, etc.

### INDUSTRIAL APPLICABILITY

As above, the liquid container of the present invention is useful as a container for an eye drop, a nose drop, a contact-lens cleaning solution or the like, and suited to obtaining a strong clicking feeling during a cap attaching operation and responding to mass-producing a container stable in accuracy, based on an injection blow molding process.

## Claims

1. A liquid container comprising: a container body (1) which has a tubular neck portion (13), a shoulder portion (11) continuous with the tubular neck portion, a liquid storage portion (10), and a nozzle (14) held by the tubular neck portion; and a top-closed tubular cap (2) attachable to the tubular neck portion (13) of the container body (1) to cover a region from the nozzle (14) to the shoulder portion (11), wherein
the tubular neck portion (13) of the container body (1) has an outer peripheral surface provided with a locking protrusion (13a) for locking the cap (2); and the cap (2) has a pair of lockable arms (24) provided on an inner top region thereof to extend along the tubular neck portion (13) and arranged radially opposite to each other, each of the pair of lockable arms (24) being formed with an engagement portion (24a) in its surface opposing to the tubular neck portion,
wherein the cap (2) is adapted to be locked with respect to the container body (1), in such a manner that, during a locking operation of attaching the cap (2) to the container body (1) along an axial direction of the tubular neck portion (13), the tubular neck portion is inserted between the oppositely arranged lockable arms (24), and the engagement portion of the lockable arms (24) is engaged with the locking protrusion (13a), and **characterized in that**:
each of the pair of the lockable arms has
a first reinforcing rib (26) formed on a back surface on an opposite side of the opposing surface and adapted to restrict a flexural displacement of the lockable arms (24) in the arrangement direction where the pair of lockable arms (24) are arranged, and
a pair of second reinforcing ribs (28) extending in the opposite directions perpendicular to the arrangement direction, the pair of second reinforcing ribs (28) connecting the lockable arm (24) and an inner surface of the cap (2).

2. The liquid container as defined in claim 1, **characterized in that** each of the engagement portions (24a) is provided in a distal end region of a corresponding one of the lockable arms (24), and
each of the first reinforcing ribs (26) is formed over a region from a base end approximately to a distal end of a corresponding one of the lockable arms (24), wherein a width of the first reinforcing rib in a direction perpendicular to a thicknesswise direction of the first reinforcing rib decreases in a direction from the base end to the distal end.

3. The liquid container as defined in claim 2, **characterized in that** each of the second reinforcing ribs (28) is provided in a vicinity of the base end of a corresponding one of the lockable arms (24).

## Patentansprüche

1. Flüssigkeitsbehälter, welcher umfasst: einen Behälterkorpus (1), der einen rohrförmigen Halsabschnitt (13), einen zu dem rohrförmigen Halsabschnitt stetigen Schulterabschnitt (11), einen Flüssigkeitsspeicherungsabschnitt (10) und eine von dem rohrförmigen Halsabschnitt gehaltene Düse (14) aufweist; und eine oben geschlossene rohrförmige Kappe (2), die an dem rohrförmigen Halsabschnitt (13) des Behälterkorpus (1) anbringbar ist, um einen Bereich von der Düse (14) zu dem Schulterabschnitt (11) abzudecken,
wobei der rohrförmige Halsabschnitt (13) des Behälterkorpus (1) eine Außenumfangsfläche aufweist, die mit einem verriegelnden Vorsprung (13a) zum Verriegeln der Kappe (2) versehen ist; und die Kappe (2) ein Paar von verriegelbaren Armen (24) aufweist, die an einem oberen Innenbereich davon vorgesehen sind, um sich entlang des rohrförmigen Halsabschnitts (13) zu erstrecken, und einander radial gegenüberliegend angeordnet sind, wobei jeder des Paars von verriegelbaren Armen (24) in seiner dem rohrförmigen Halsabschnitt gegenüberliegenden Fläche mit einem Eingriffabschnitt (24a) ausgebildet ist,
wobei die Kappe (2) ausgelegt ist, um bezüglich des Behälterkorpus (1) so verriegelt zu werden, dass während eines Verriegelungsvorgangs des Anbringens der Kappe (2) an dem Behälterkorpus (1) entlang einer axialen Richtung des rohrförmigen Halsabschnitts (13) der rohrförmige Halsabschnitt zwischen den gegenüber angeordneten verriegelbaren Armen (24) eingesetzt wird und der Eingriffabschnitt der verriegelbaren Arme (24) mit dem verriegelnden Vorsprung (13a) in Eingriff gebracht wird, und
**dadurch gekennzeichnet, dass** jeder des Paars der verriegelbaren Arme aufweist:
eine erste Verstärkungsrippe (26), die an einer Rückfläche an einer gegenüberliegenden Seite der gegenüberliegenden Fläche ausgebildet und ausgelegt ist, um eine biegende Verlagerung der verriegelnden Arme (24) in der Anordnungsrichtung, in der das Paar von verriegelbaren Armen (24) angeordnet sind, zu beschränken, und
ein Paar von zweiten Verstärkungsrippen (28), die sich in den Gegenrichtungen senkrecht zu der Anordnungsrichtung erstrecken, wobei das Paar von zweiten Verstärkungsrippen (28) den verriegelbaren Arm (24) und eine Innenfläche der Kappe (2) verbindet.

2. Flüssigkeitsbehälter nach Anspruch 1, **dadurch gekennzeichnet, dass** jeder der Eingriffabschnitte (24a) in einem distalen Endbereich eines entsprechenden der verriegelbaren Arme (24) vorgesehen ist und
jede der ersten Verstärkungsrippen (26) über einem Bereich von einem Basisende in etwa zu einem distalen Ende eines entsprechenden der verriegelbaren Arme (24) ausgebildet ist, wobei eine Breite der ersten Verstärkungsrippe in einer Richtung senkrecht zu einer Dickenrichtung der ersten Verstärkungsrippe in einer Richtung von dem Basisende zu dem distalen Ende abnimmt.

3. Flüssigkeitsbehälter nach Anspruch 2, **dadurch gekennzeichnet, dass** jede der zweiten Verstärkungsrippen (28) in einer Nähe des Basisendes eines entsprechenden der verriegelbaren Arme (24) vorgesehen ist.

## Revendications

1. Récipient à liquide comprenant : un corps de récipient (1) qui présente une partie de goulot tubulaire (13), une partie d'épaulement (11) continue avec la partie de goulot tubulaire, une partie de stockage de liquide (10), et un embout (14) maintenu par la partie de goulot tubulaire ; et un bouchon tubulaire (2) fermé en haut pouvant être relié à la partie de goulot tubulaire (13) du corps de récipient (1) pour recouvrir une zone allant de l'embout (14) à la partie d'épaulement (11),
dans lequel la partie de goulot tubulaire (13) du corps de récipient (1) présente une surface périphérique extérieure dotée d'une saillie de verrouillage (13a) pour verrouiller le bouchon (2) ; et le bouchon (2) présente une paire de bras verrouillables (24) prévus sur une zone supérieure intérieure de celui-ci pour s'étendre le long de la partie de goulot tubulaire (13) et agencés opposés radialement l'un à l'autre, chacun de la paire de bras verrouillables (24) étant formé avec une partie d'engagement (24a) dans sa surface faisant face à la partie de goulot tubulaire,
dans lequel le bouchon (2) est adapté pour être verrouillé par rapport au corps de récipient (1) de telle façon que, pendant une opération de verrouillage consistant à relier le bouchon (2) au corps de récipient (1) le long d'une direction axiale de la partie de goulot tubulaire (13), la partie de goulot tubulaire est insérée entre les bras verrouillables (24) agencés opposés l'un à l'autre, et la partie d'engagement des bras verrouillables (24) est engagée dans la saillie de verrouillage (13a), et
**caractérisé en ce que** :
chacun de la paire de bras verrouillables présente
une première nervure de renforcement (26) formée sur une surface arrière sur une face opposée de la surface faisant face et adaptée pour limiter un déplacement de flexion des bras verrouillables (24) dans la direction d'agencement dans laquelle la paire de bras verrouillables (24) est agencée, et
une paire de secondes nervures de renforcement (28) s'étendant dans les directions opposées perpendiculaires à la direction d'agencement, la paire de secondes nervures de renforcement (28) reliant le bras verrouillable (24) et une surface intérieure du bouchon (2).

2. Récipient à liquide selon la revendication 1, **caractérisé en ce que** chacune des parties d'engagement (24a) est prévue dans une zone d'extrémité distale d'un bras correspondant des bras verrouillables (24), et
chacune des premières nervures de renforcement (26) est formée sur une zone allant d'une extrémité de base approximativement à une extrémité distale d'un bras correspondant des bras verrouillables (24), dans lequel une largeur de la première nervure de renforcement dans une direction perpendiculaire à une direction dans le sens de l'épaisseur de la première nervure de renforcement diminue dans une direction allant de l'extrémité de base à l'extrémité distale.

3. Récipient à liquide selon la revendication 2, **caractérisé en ce que** chacune des secondes nervures de renforcement (28) est prévue à proximité de l'extrémité de base d'un bras correspondant des bras verrouillables (24).
